Europäisches Patentamt

European Patent Office

Office européen des brevets

(19)

(11) Veröffentlichungsnummer: **0 250 409**
**B1**

(12) **EUROPÄISCHE PATENTSCHRIFT**

(45) Veröffentlichungstag der Patentschrift: **09.05.90**

(51) Int. Cl.⁵: **A 61 F 13/10**

(21) Anmeldenummer: **86901336.7**

(22) Anmeldetag: **08.02.86**

(86) Internationale Anmeldenummer:
**PCT/DE86/00046**

(87) Internationale Veröffentlichungsnummer:
**WO 86/04811 28.08.86 Gazette 86/19**

(54) **EPICONDYLITIS-BANDAGE.**

(30) Priorität: **14.02.85 DE 8504357 u**

(43) Veröffentlichungstag der Anmeldung:
**07.01.88 Patentblatt 88/01**

(45) Bekanntmachung des Hinweises auf die
Patenterteilung:
**09.05.90 Patentblatt 90/19**

(84) Benannte Vertragsstaaten:
**AT BE CH DE FR GB IT LI LU NL SE**

(56) Entgegenhaltungen:
**EP-A-0 086 960**
**GB-A-2 074 452**
**US-A-3 322 118**
**US-A-3 970 081**
**US-A-4 128 097**
**US-A-4 441 493**

(73) Patentinhaber: **Weihermüller & Voigtmann
GmbH & Co. KG
Waldsteinring 10
D-8580 Bayreuth-St. Johannis (DE)**

(72) Erfinder: **BAUMGARTNER, Sepp
Zwieselstr. 24
D-8221 Waging am See (DE)**
Erfinder: **WEIHERMÜLLER, Wolfgang
Waldsteinring 12
D-8580 Bayreuth 24 (DE)**
Erfinder: **VOIGTMANN, Günter
Waldsteinring 10
D-8580 Bayreuth 24 (DE)**

(74) Vertreter: **Voigt, Günter, Dipl.-Ing.
Patentanwälte Dr.-Ing. Alfred Schulze, Dipl.-Ing.
Günter Voigt & Partner Nordring 152, Postfach
210104
D-8500 Nürnberg 21 (DE)**

## Beschreibung

Die Erfindung bezieht sich auf eine Epicondylitis-Bandage gemäß Oberbegriff des Anspruchs 1.

Eine solche Bandage dient zur Behandlung eines "Tennisarms". Bei einem sogenannten "Tennisarm" handelt es sich um einen schmerzhaften Reizzustand im Ursprungsbereich der Unterarmstrecksehnen an der Ellbogenaußenseite, dort wo die Sehnen am Knochen verankert sind. Anders als die Bezeichnung "Tennisarm" vermuten läßt, treten solche schmerzhaften Reizzustände aber nicht nur bei Tennisspielern auf, sondern stellen einen schmerzhaften Reizzustand dar, der zahlenmäßig in noch größerem Umfang auch bei beruflichen Tätigkeiten oder Tätigkeiten im Haushalt festzustellen ist.

Bei Tennisspielern treten solche Reizzustände auf, wenn ein Tennisspieler eine falsche Technik, d.h. zu viel Kraft statt Schwung, anwendet. Oft sind auch Metallschläger die Ursache für einen sogenannten Tennisarm. Sie absorbieren die Schwingungen weniger als traditionelle Holz- oder moderne Corbon-Fiber-Schläger. Insbesondere derjenige, der seinen Schläger knallhart bespannt, setzt seinen Arm großer Gefahr aus. Wird dann der Ball auch nur ein paarmal in der Stunde falsch getroffen, kann dies die Ursache für Beschwerden sein. Auch das Wechseln vom langsamen Grundplatz auf schnelle Böden wie Beton, Kunststoff, Kunstrasen oder Teppich fördert die Beschwerden. Der Ball springt anders, fast immer schneller. Die ballistische Kurve wird flacher und der Ball ist schlechter zu berechnen. Dadurch kommt es zu überhasteten, unharmonischen Bewegungen. Handgelenk und Unterarmmuskulatur müssen in Sekundenbruchteilen verkrampft die fehlerhaften Schlagtechniken ausgleichen.

Ganz Entsprechendes läßt sich auch feststellen, wenn jemand im Beruf bestimmte krafterfordernde Bewegungen überhastet und unharmonisch ausführt. Dies ist insbesondere dann der Fall, wenn ein bestimmter Zeitrhythmus von außen vorgegeben wird, insbesondere also bei Band- oder Akkordarbeit.

Aber auch bei im Haushalt oder im Büro tätigen Frauen kann der sogenannte "Tennisarm" auftreten. Bügeln, Stricken oder Maschineschreiben können in solchen Fällen die Ursache sein.

Im akuten Zustand der Reizung bereitet schon das Heben einer Kaffeetasse Schmerzen. Darüber hinaus ist die schmerzhafte Stelle druckempfindlich.

Zur Behandlung werden u.a. verschiedenartige Salbenverbände empfohlen. Es ist auch bereits eine sogenannte Epicondylitis-Spange bekannt geworden, die den Arm in der Nähe des Ellbogengelenks im wesentlichen halbringförmig umspannt (DE—PS 26 35 426). Ein solches riemenähnliches Gebilde hat jedoch den Nachteil, daß praktisch kein Wärmeeffekt ermöglicht wird und die Druckpolster nicht gelockert werden können, da die sonst zu einem Verrutschen der Druckpolster führen würde. Es hat sich jedoch als vorteilhaft erwiesen, den Druck der Platten nach ca. 10 Minuten Betätigung im gewissen Umfang zu lockern, da die Muskulatur bei einer Belastung wegen der stärkeren Durchblutung bekanntlich anschwillt und dem durch die Lockerung der Zugbänder im ausreichenden Maße Rechnung getragen werden muß. Anderenfalls kommt es zu einer raschen Ermüdung und Erlahmung der Muskulatur. Schließlich geben bei der vorbekannten Ausführung die Druckpolster den Druck nur wenig konzentriert weiter. Der erzielbare Effekt wird dadurch gemindert.

Aus der US—PS 3 970 081 ist eine elastische schlauchförmige Bandage bekannt, die das Ellbogengelenk umfaßt und die ein elastisches (sogenanntes) Druckkissen aufweist, das mittels eines Spannriemens auf einen der Epicondylen gedrückt wird.

Aus der US—PS 3 322 118 ist eine aus Baumwollfasern oder ähnlichen hergestellte saugfähige Bandage bekannt, die mit einem aus Gummi bestehenden Kissen versehen ist, das seinerseits mit den Faser der Bandage verbunden ist.

Aus der US—PS 4 441 493 schließlich ist ein relativ schmaler Stützriemen mit einem elastischen Druckkissen bekannt, aus auf einen der Epicondylen einwirkt.

Bei den zuletzt genannten Epicondylitis-Bandagen gibt es nur eine Druckplatte und es fehlt nicht nur der erwünschte sichere Halt der Druckplatten auch nach einer Lockerung des Spannriemen, sondern es fehlt darüber hinaus auch die therapeutisch erwünschte, zu beiden Seiten um das Ellbogengelenk im wesentlichen gleichmäßig verteilte Wirkung der Wärme, da nur ein sehr schmaler Streifen den Arm umfaßt.

Der Erfindung liegt die Aufgabe zugrunde, eine Epicondylitis-Bandage zu schaffen, die sowohl aus medizinischer Sicht als auch im Hinblick auf ihre praktische Anwendung von besonderem Vorteil ist.

Dies geschieht mit Hilfe der kennzeichnenden Merkmale des Anspruchs 1.

Vorteilhafte Weiterbildungen ergeben sich aus den Unteransprüchen.

Von Bedeutung ist dabei, daß die Druckwirkung durch einen den Arm umgreifenden Riemen mit Hilfe von Plättchen erzeugt wird, die den Positionen der Epicondylen zugeordnet sind. Riemen und Plättchen sind fest mit einer elastischen Armbandage verbunden. Die Armbandage umschließt einen Teil des Unterarms, Ellbogen und einen Teil des Oberarms und ist im höchsten Maße rutschfest und garantiert die medizinisch richtige Lage von Spannriemen mit Plättchen auch bei nur geringer Spannung des Spannriemens. Ein weiterer Vorteil ist die Wärmewirkung der Bandage.

Die Erfindung wird nachfolgend unter Bezugnahme auf die Zeichnungsfigur beispielsweise erläutert.

In der Zeichnungsfigur ist ein aus elastischem Material gearbeiteter, insbesondere gestrickter oder rundgestrickter Schlauchabschnitt 10 zu erkennen, der in seiner Form im wesentlichen dem Armgelenk angepaßt ist. Der Schlauchabschnitt 10 kann so gearbeitet sein, daß die in Umfangsrichtung vorhandene elastische Spannung in Längsrichtung gesehen vom Unterarm zum Oberarm hin kontinuierlich abnimmt. Dies wirkt sich vorteilhaft auf die Durchblutung aus.

Der Schlauchabschnitt 10 ist so gestaltet, daß er in seiner Form den anatomischen Verhältnissen um das Ellbogengelenk entspricht. An der dem Ellbogengelenk zugeordneten Stelle des Schlauchabschnitts 10 kann ein Zwickel 11 eingenäht sein oder aber der Schlauchabschnitt in einem kontinuierlichen Arbeitsvorgang, beispielsweise beim Rundstrickvorgang, eine leichte Ausbauchung erhalten haben.

Wird der Schlauchabschnitt 10 nicht rundgestrickt, sondern aus einer Flachbahn oder aus einem Flachgestrick hergestellt, so wird er eine Naht aufweisen, die vorzugsweise an der der Innenseite des Arms entsprechenden Längslinie des Schlauchabschnitts 10 verlaufen sollte. An den den Epicondylen (Nervus radialis profundus) entsprechenden Stellen des Schlauchabschnitts 10 sind relativ feste, im wesentlichen plattenförmige Einlagen 12 vorgesehen, die an diesen Stellen — ggf. in einer Tasche — auf den Schlauchabschnitt 10 genäht oder aber anderweitig dort befestigt sind. Dadurch wird in erster Linie eine mechanische Entlastung der Muskeln an ihren Ansatzpunkten, nämlich an den Epicondylen, erreicht. Die Folge davon wiederum ist eine Entspannung der Hand- und Fingerstreckmuskulatur und demzufolge eine Besserung der Knochenhautreizung. Die als Druckplatte dienende Einlage 12 ist vorzugsweise eben und besteht aus einem elastisch verformbaren Material. Sie wird durch den elastischen Schlauchabschnitt 10 und durch den Spannriemen 13 an den Radius des Unterarms angepaßt. Dabei ist der Druck der Platte in deren Mitte verständlicherweise am größten und nimmt zum Randbereich hin kontinuierlich ab. So wird der Druck sehr gezielt auf den Nervus radialis profondus konzentriert, der für die Wahrnehmung der Schmerzen beim sogenannten "Tennisarm" verantwortlich ist. Der Druck führt zu einer Beruhigung und Schmerzlinderung. Insbesondere bei hartelastischen Druckplatten konzentriert sich der Druck stark auf deren Mitte.

Wie bereits weiter oben erwähnt, wird durch den verstärkten Druck der Platten auf die Unterarmmuskulatur am Epicondylus lateralis und medialis eine Zugentlastung an den Sehnenansätzen (Extensoren und Flexoren) erreicht. Weiter wird durch die Platten eine zusätzliche Dämpfung des Aufprallschocks und der Schwingungen vom Schläger auf den Sehnenansatz ermöglicht. Schmerzhafte Druckstellen am Randbereich der Platten werden dadurch vermieden, daß der Druck zum Randbereich hin praktisch kontinuierlich abnimmt.

Über einen Spannriemen 13, der die beiden vorgesehenen plättchenförmigen Einlagen 12 zumindest teilweise übergreift, kann die Umfangsspannung in diesem Bereich eingestellt werden. Um eine stufenlose, den jeweiligen Bedürfnissen entsprechende Verstellung der Spannung des Spannriemens 13 zu erreichen, ist dieser mit einem an sich bekannten Verschluß versehen. Dabei kann es sich um einen Riemen- oder Schnallenverschluß, aber auch um einen sogenannten Klettverschluß handeln.

Der Spannriemen 13 kann auch doppelt geführt werden, indem er durch eine Öse 14 und von dort in seine Ausgangsrichtung zurückgeführt wird. Die Fixierung des Spannriemens 13 erfolgt dann gegenüber dem Spannriemen 13 selbst, wie dies in der Zeichnungsfigur schematisch dargestellt ist.

Die oben geschilderte Epicondylitis-Bandage hat sich in der Praxis außerordentlich gut bewährt. Dies dürfte auch darauf zurückzuführen sein, daß durch den elastischen Schlauchabschnitt 10 in Verbindung mit den plattenförmigen Einlagen 12 eine mechanische Entlastung der Muskeln in ihren Ansatzpunkten, nämlich den Epicondylen, erreicht wird, wodurch sich eine Entspannung der Hand- und Fingerstreckmuskulatur ergibt. Ein weiterer Vorteil ist die zusätzliche Wärmewirkung der Bandage, insbesondere in den Fällen, in denen eine wärmeisolierende textile oder andere Lage zusätzlich vorgesehen ist oder aber ein zusätzliches wärmeisolierendes Fadenmaterial in das Gewebe oder Gestrick aufgenommen worden ist.

Die oben beschriebene Bandage kann auch prophylaktisch bei arbeits- oder sportbedingten Bewegungen zum Einsatz kommen, die möglicherweise eine Epicondylitis auslösen oder aber bereits einmal zu einer ersten Erkrankung geführt haben. Insbesondere nach einer ersten Erkrankung besteht stets die Gefahr eines Rezidivs.

Die gezielte Druckentwicklung, radial und/oder ulnar, führt im gefährdeten Bereich zu einer optimalen Druckverteilung. Der elastische Schlauchabschnitt 10 entspricht der anatomischen Form des Unterarms und garantiert Rutschfestigkeit, optimalen Sitz und ggf. auch eine kontinuierliche Veränderung der Umfangskräfte über die Länge des Schlauchabschnitts 10.

Der nicht vom Spannriemen 13 erfaßte Umfangsabschnitt kann mit einem elastischen oder auch weitgehend unelastischen Band 15 besetzt sein, das auch dann für einen gewissen Mindestzug in Umfangsrichtung sorgt, wenn der Anwender nach einer gewissen Zeit der Tätigkeit den Spannriemen 13 nach und nach lockert. Dabei ermöglicht der Verschluß eine individuelle Anpassung der Spannung des Spannriemens 13, insbesondere bei Verwendung des Klettverschlusses.

Zu erwähnen ist noch, daß der oben beschriebene Schlauchabschnitt 10 zu einer Verstärkung der Venenpumpe im gesamten Unterarmbereich führt, und zwar ohne Beeinträchtigung der arteriellen Durchblutung. Die Größe der plattenförmigen Einlagen 12 kann der Größe des Arms in gewissen Umfang angepaßt werden, wird im Durchschnitt bei etwa 7,5 cm × 5,5 cm liegen.

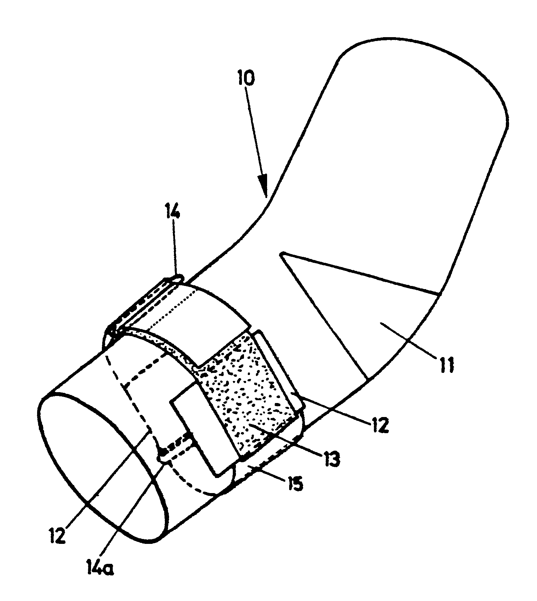

**Patentansprüche**

1. Epicondylitis-Bandage, im wesentlichen bestehend aus einem Schlauchabschnitt (10) aus elastischem Material, wobei die Zugspannung in Umfangsrichtung über einen im wesentlichen in Umfangsrichtung verlaufenden Spannriemen (13) mit Verschluß veränderbar ist, dadurch gekennzeichnet, daß sich der erwähnte Schlauchabschnitt (10) im wesentlichen gleichmäßig zu beiden Seiten um das Ellbogengelenk erstreckt, daß es sich bei dem elastischen Material des Schlauchabschnitts (10) um ein Gestrick mit wärmedämmender Wirkung handelt sowie daß an den den Epicondylen entsprechenden Stellen im wesentlichen plattenförmige hartelastische Einlagen (12) vorhanden sind, wobei der Spannriemen (13) zwischen den erwähnten Einlagen (12) diese zumindest teilweise übergreifend angeordnet ist.

2. Bandage nach Anspruch 1, dadurch gekennzeichnet, daß der Schlauchabschnitt (10) an der dem Ellbogengelenk zugeordneten Stelle einen eingearbeiteten Zwickel (11) aufweist.

3. Bandage nach Anspruch 1, dadurch gekennzeichnet, daß der Schlauchabschnitt (10) an der dem Armgelenk zugeordneten Stelle eine eingearbeitete leichte Ausbauchung aufweist.

4. Bandage nach einem der Ansprüche 1 bis 3, dadurch gekennzeichnet, daß der Spannriemen (13) teilweise innerhalb des Schlauchabschnitts (10) angeordnet ist, der Verschluß des Spannriemens (13) sich jedoch außerhalb des Schlauchabschnitts (10) befindet.

5. Bandage nach einem der Ansprüche 1 bis 3, dadurch gekennzeichnet, daß der Spannriemen (13) den Umfang des Schlauchabschnitts (10) außen umgreift.

6. Bandage nach einem der Ansprüche 1 bis 5, dadurch gekennzeichnet, daß die plattenförmigen Einlagen (12) in einer oder mehreren taschenförmigen Elementen angeordnet sind.

7. Bandage nach einem der Ansprüche 1 bis 6, dadurch gekennzeichnet, daß der nicht vom Spannriemen (13) abgedeckte Umfangsabschnitt als elastisches Band (15) ausgebildet ist.

8. Bandage nach einem der Ansprüche 1 bis 7, dadurch gekennzeichnet, daß der Schlauchabschnitt (10) vorzugsweise innen, eine wärmedämmende textile oder andere Lage aufweist.

9. Bandage nach einem der Ansprüche 1 bis 7, dadurch gekennzeichnet, daß der Schlauchabschnitt (10) ein aus elastischen und aus wärmeisolierenden Fäden gebildetes Gestrick oder Gewebe ist.

10. Bandage nach Anspruch 1 oder einem der Ansprüche 3 bis 9, dadurch gekennzeichnet, daß der Schlauchabschnitt (10) rundgestrickt, also nahtlos ist.

11. Bandage nach einem der Ansprüche 1 bis 9, dadurch gekennzeichnet, daß die Naht des Schlauchabschnitts (10) an der der Innenseite des Arms zugeordneten Längslinie des Schlauchabschnitts (10) angeordnet ist.

12. Bandage nach einem der Ansprüche 1 bis 11, dadurch gekennzeichnet, daß es sich bei den Einlagen (12) um Kunststoff-Plättchen handelt.

13. Bandage nach einem der Ansprüche 1 bis 11, dadurch gekennzeichnet, daß es sich bei den Einlagen (12) um Metall-Plättchen handelt.

14. Bandage nach einem der Ansprüche 1 bis 11, dadurch gekennzeichnet, daß es sich bei den Einlagen (12) um Holz-Plättchen handelt.

15. Bandage nach einem der Ansprüche 1 bis 14, dadurch gekennzeichnet, daß der Spannriemen (13) durch eine Öse (14) und von dort in seine Ausgangsrichtung zurückgeführt ist.

16. Bandage nach einem der Ansprüche 1 bis 15, dadurch gekennzeichnet, daß der Spannriemen (13) mit einem Schnallenverschluß versehen ist.

17. Bandage nach einem der Ansprüche 1 bis 15, dadurch gekennzeichnet, daß der Spannriemen (13) mit einem Klettenverschluß versehen ist.

**Revendications**

1. Bandage épicondylien constitué pour l'essentiel d'un segment tubulaire (10) en matériau élastique, l'effort de tension périphérique étant réglable par une ceinture tendeuse (13) pourvue d'une fermeture et entourant ledit segment tubulaire, caractérisé en ce que:

le segment tubulaire (10) s'étend à peu près régulièrement de part et d'autre du coude,

le matériau élastique constituant ledit segment tubulaire (10) est un tricot à effet d'isolation thermique,

aux endroits correspondant à l'emplacement des épicondyles sont prévues des garnitures (12) sensiblement en forme de plaquettes à élasticité ferme, la ceinture tendeuse (13) disposée entre lesdites garnitures recouvrant celles-ci au moins partiellement.

2. Bandage selon la revendication 1 caractérisé en ce que le segment tubulaire (10) comporte à l'emplacement correspondant au coude, un gousset (11) travaillé dans la pièce.

3. Bandage selon la revendication 1 caractérisé en ce que le segment tubulaire (10) comporte à l'emplacement correspondant à l'articulation du bras, une légère convexité travaillée dans la pièce.

4. Bandage selon l'une des revendications 1 à 3 caractérisé en ce que le ceinture tendeuse (13) est disposée partiellement à l'intérieur du segment tubulaire (10), sa fermeture étant prévue à l'extérieur dudit segment tubulaire.

5. Bandage selon l'une des revendications 1 à 3 caractérisé en ce que la ceinture tendeuse (13) entoure le segment tubulaire (10) extérieurement.

6. Bandage selon l'une des revendications 1 à 5 caractérisé en ce que les garnitures (12) en forme de plaquettes sont disposées dans un ou plusieurs éléments en forme de poches.

7. Bandage selon l'une des revendications 1 à 6 caractérisé en ce que la partie de la surface périphérique qui n'est pas couverte par le ceinture tendeuse (13) est constituée d'un bandeau élastique.

8. Bandage selon l'une des revendications 1 à 7 caractérisé en ce que le segment tubulaire (10) comporte de préférence intérieurement une couche textile ou autre à effet d'isolation thermique.

9. Bandage selon l'une des revendications 1 à 7 caractérisé en ce que le segment tubulaire (10) est un tricot ou tissu fabriqué à partir de fils élastiques et de fils à effet d'isolation thermique.

10. Bandage selon la revendication 1 ou l'une des revendications 3 à 9 caractérisé en ce que le segment tubulaire (10) est tricoté en rond et ne comporte pas de couture.

11. Bandage selon l'une des revendications 1 à 9 caractérisé en ce que la couture du segment tubulaire (10) est disposée longitudinalement, du côté intérieur du bras.

12. Bandage selon l'une des revendications 1 à 11 caractérisé en ce que les garnitures (12) sont des plaquettes en matière synthétique.

13. Bandage selon l'une des revendications 1 à 11 caractérisé en ce que les garnitures (12) sont des plaquettes métalliques.

14. Bandage selon l'une des revendications 1 à 11 caractérisé en ce que les garnitures (12) sont des plaquettes de bois.

15. Bandage selon l'une des revendications 1 à 14 caractérisé en ce que la ceinture tendeuse (13) passe à travers une bride (15) à partir de laquelle elle est retournée en direction de son point de départ.

16. Bandage selon l'une des revendications 1 à 15 caractérisé en ce que la ceinture tendeuse (13) comporte une fermeture à boucle.

17. Bandage selon l'une des revendications 1 à 15 caractérisé en ce que la ceinture tendeuse (13) comporte une fermeture à crampons.

**Claims**

1. Epicondylitis bandage, consisting essentially of a tubular section (10) made of elastic material, the tension in the circumferential direction being changeable via a tension strap (13), extending essentially in the circumferential direction, with a fastening, characterized in that the mentioned tubular section (10) extends essentially evenly on both sides around the elbow joint, in that the elastic material of the tubular section (10) is a knitted fabric with thermal insulating effect, and in that essentially plate-shaped hard elastic inserts (12) are present at the points corresponding to the epicondyles, the tension strap (13) being arranged between the mentioned inserts (12) to overlap the latter at least partially.

2. Bandage according to Claim 1, characterized in that the tubular section (10) has an integrated gusset (11) at the point allocated to the elbow joint.

3. Bandage according to Claim 1, characterized in that the tubular section (10) has an integrated, slight bulge at the point allocated to the arm joint.

4. Bandage according to one of Claims 1 to 3, characterized in that the tension strap (13) is arranged partially inside the tubular section (10), the fastening of the tension strap (13), however, being situated outside the tubular section (10).

5. Bandage according to one of Claims 1 to 3, characterized in that the tension strap (13) surrounds the circumference of the tubular section (10) on the outside.

6. Bandage according to one of Claims 1 to 5, characterized in that the plate-shaped inserts (12) are arranged in one or more pocket-shaped elements.

7. Bandage according to one of Claims 1 to 6, characterized in that the circumferential section not covered by the tension strap (13) is constructed as an elastic strip (15).

8. Bandage according to one of Claims 1 to 7, characterized in that the tubular section (10) has a thermal insulating textile or other layer, preferably on the inside.

9. Bandage according to one of Claims 1 to 7, characterized in that the tubular section (10) is a knitted or woven fabric made of elastic and of thermal insulating threads.

10. Bandage according to Claim 1 or one of Claims 3 to 9, characterized in that the tubular section (10) is circular-knitted, that is to say seamless.

11. Bandage according to one of Claims 1 to 9, characterized in that the seam of the tubular section (10) is arranged on the longitudinal line of the tubular section (10) allocated to the inside of the arm.

12. Bandage according to one of Claims 1 to 11, characterized in that the inserts (12) are small plastic plates.

13. Bandage according to one of Claims 1 to 11, characterized in that the inserts (12) are small metal plates.

14. Bandage according to one of Claims 1 to 11, characterized in that the inserts (12) are small wooden plates.

15. Bandage according to one of Claims 1 to 14, characterized in that the tension strap (13) is guided through an eyelet (14) and returned from there in its starting direction.

16. Bandage according to one of Claims 1 to 15, characterized in that the tension strap (13) is provided with a buckle fastening.

17. Bandage according to one of Claims 1 to 15, characterized in that the tension strap (13) is provided with a touch-and-close fastening.